# EUROPEAN PATENT APPLICATION

(11) **EP 4 763 272 A1**
(43) Date of publication of application: **24.06.2026**
(21) Application number: 24887752.4
(22) Date of filing: 23.10.2024
(51) Int. Cl.: A61N 1/05, A61N 1/36, A61B 5/262, A61B 5/294, A61B 5/388, A61B 5/00

(54) **IMPLANTABLE ELECTRODE, STIMULATOR AND CONTROL METHOD THEREFOR, ELECTRONIC DEVICE, AND STORAGE MEDIUM**

(30) Priority: 06.11.2023 CN 202311461723
(71) Applicant: Sceneray Co., Ltd., Suzhou, Jiangsu 215000 (CN)
(72) Inventor: ZHI, Menghui, Suzhou, Jiangsu 215000 (CN); ZHU, Weiran, Suzhou, Jiangsu 215000 (CN)
(74) Representative: Cabinet Beaumont
(86) International application number: PCT/CN2024/126711
(87) International publication number: WO 2025/098139

(57) **Abstract**

Provided are an implantable electrode, a stimulator and a control method therefor, an electronic device, and a storage medium. The implantable electrode includes a control module, an array switch, and an electrode array module. The control module is configured to receive a gating instruction from a pulse generator and generate a switch control signal. The array switch is configured to establish multiple gated channels according to the switch control signal. The electrode array module includes multiple contacts. Each contact is configured to deliver electrical stimulation to tissue of a patient through a gated channel established by the array switch or configured to collect a physiological signal from tissue of a patient through a gated channel established by the array switch. The switch control signal is used for isolating the stimulation contact from the collection contact.

## Description

This application claims priority to Chinese Patent Application No. 202311461723.7 filed with the China National Intellectual Property Administration (CNIPA) on Nov. 6, 2023, the disclosure of which is incorporated herein by reference in its entirety.

### TECHNICAL FIELD

The present application relates to the technical field of implantable medical devices, for example, an implantable electrode, a stimulator and a control method therefor, an electronic device, and a computer-readable storage medium.

### BACKGROUND

A stimulator is an implantable medical device. A stimulator includes an (implantable) pulse generator, an extension lead, and an electrode. A stimulator can provide a patient with finely-tuned parameter-controllable electrical stimulation.

The electrode includes multiple contacts. For real-time closed-loop stimulation, the contacts on the electrode are required to perform signal collection in addition to electrical stimulation. In other words, the electrode serves as a carrier for electrical stimulation therapy and signal collection. In general, the amplitude of a collected signal is on the order of microvolts (µV) while the amplitude of the electrical stimulation is on the order of volts (V). The difference between the two magnitudes makes the collected signal susceptible to interference from the electrical stimulation, thereby affecting parsing of the collected signal.

### SUMMARY

The present application provides an implantable electrode, a stimulator and a control method therefor, an electronic device, and a computer-readable storage medium to meet the requirements of practical application.

The present application provides an implantable electrode. The implantable electrode includes a control module, an array switch, and an electrode array module.

The control module is configured to receive a gating instruction from a pulse generator and generate a switch control signal.

The array switch is configured to establish multiple gated channels according to the switch control signal.

The electrode array module includes multiple contacts. Each contact is configured as a stimulation contact to deliver electrical stimulation to tissue of a patient through a gated channel established by the array switch or configured as a collection contact to collect a physiological signal from tissue of a patient through a gated channel established by the array switch.

The switch control signal is used for isolating the stimulation contact for delivering the electrical stimulation from the collection contact for collecting the physiological signal.

In some possible implementations, the switch control signal includes a high-level signal and a low-level signal, the high-level signal is used for gating a channel of the array switch, and the low-level signal is used for disconnecting a channel of the array switch.

In some possible implementations, the array switch includes multiple switch modules.

Each switch module includes an insulating layer, a switch unit, and a conductive layer.

The insulating layer is configured to form an accommodation cavity.

The switch unit is disposed in the accommodation cavity formed by the insulating layer and configured to establish, according to the switch control signal, a gated channel of a contact corresponding to the switch control signal.

The conductive layer coats the outer surface of the insulating layer and is configured to be grounded through a wire to shield at least one of electric-field interference or electromagnetic interference.

In some possible implementations, the electrode array module is a Utah electrode.

In some possible implementations, the physiological signal is a local field potential signal.

In a second aspect, the present application provides a stimulator. The stimulator includes any preceding implantable electrode and a pulse generator.

The implantable electrode is configured to deliver the electrical stimulation to the tissue of the patient and collect the physiological signal from the tissue of the patient.

The pulse generator is electrically connected to the implantable electrode directly or through an extension lead and configured to parse the physiological signal and generate the electrical stimulation.

In some possible implementations, the pulse generator includes a stimulation contact determination module, an isolation contact acquisition module, and a collection contact determination module.

The stimulation contact determination module is configured to use at least one contact in the electrode array module as at least one stimulation contact according to therapy information of the patient. Each stimulation contact is configured to deliver the electrical stimulation to the tissue of the patient.

The isolation contact acquisition module is configured to acquire, for each stimulation contact, multiple contacts as isolation contacts. The multiple contacts satisfy at least one of a preset distance from the stimulation contact or a preset position relationship with the stimulation contact.

The collection contact determination module is configured to select a contact from contacts other than the at least one stimulation contact and the isolation contacts of each stimulation contact according to the therapy information to serve as a collection contact; and, when the stimulation contact delivers the electrical stimulation to the tissue of the patient, collect the physiological signal from the tissue of the patient by using the collection contact and take the collected physiological signal as collection information.

In some possible implementations, the therapy information includes a preset parameter corresponding to each stimulation contact. The preset parameter indicates a distance relationship or a position relationship between the stimulation contact and an isolation contact corresponding to the stimulation contact.

In some possible implementations, the isolation contact acquisition module includes a parameter prediction unit, a similarity determination unit, a first contact acquisition unit, and a second contact acquisition unit.

The parameter prediction unit is configured to input a stimulation parameter corresponding to the stimulation contact into a prediction model to acquire a reference preset parameter corresponding to the stimulation contact.

The similarity determination unit is configured to acquire a first similarity between the reference preset parameter and the preset parameter.

The first contact acquisition unit is configured to, in response to the first similarity being less than a preset similarity, perform an eight-neighborhood search on the stimulation contact based on the preset parameter to acquire the isolation contacts.

The second contact acquisition unit is configured to, in response to the first similarity being not less than the preset similarity, update the preset parameter by using the reference preset parameter and perform the eight-neighborhood search on the stimulation contact based on the updated preset parameter to acquire the isolation contacts.

In some possible implementations, the pulse generator also includes a counting module and a risk prompt module.

The counting module is configured to, in response to the first similarity being less than the preset similarity, add one to a count calculated by the counting module and determine whether the count added by one is greater than a preset count.

The risk prompt module is configured to, in response to the count added by one being greater than the preset count, generate risk prompt information and clear the count added by one.

In some possible implementations, the pulse generator also includes a recommended-contact acquisition module.

The recommended-contact acquisition module is configured to determine the score of each stimulation contact according to a stimulation parameter and collection information corresponding to each stimulation contact and use the stimulation contact with the highest score as a recommended stimulation contact.

In a third aspect, the present application provides a control method of a stimulator. The control method is applicable to any preceding stimulator. The control method includes:
using at least one contact in an electrode array module as at least one stimulation contact according to therapy information of a patient, where each stimulation contact is configured to deliver electrical stimulation to tissue of the patient;
acquiring, for each stimulation contact, multiple contacts as isolation contacts, where the multiple contacts satisfy at least one of a preset distance from the stimulation contact or a preset position relationship with the stimulation contact; and
selecting a contact from contacts other than the at least one stimulation contact and the isolation contacts of each stimulation contact according to the therapy information to serve as a collection contact; and, when the stimulation contact delivers the electrical stimulation to the tissue of the patient, collecting a physiological signal from the tissue of the patient by using the collection contact and taking the collected physiological signal as collection information.

In some possible implementations, the therapy information includes a preset parameter corresponding to each stimulation contact. The preset parameter indicates a distance relationship or a position relationship between the stimulation contact and an isolation contact corresponding to the stimulation contact.

In some possible implementations, acquiring, for each stimulation contact, multiple contacts as isolation contacts by an eight-neighborhood search, where the multiple contacts satisfy at least one of a preset distance from the stimulation contact or a preset position relationship with the stimulation contact, includes:
inputting a stimulation parameter corresponding to the stimulation contact into a prediction model to acquire a reference preset parameter corresponding to the stimulation contact;
acquiring a first similarity between the reference preset parameter and the preset parameter;
in response to the first similarity being less than a preset similarity, performing an eight-neighborhood search on the stimulation contact based on the preset parameter to acquire the isolation contacts; and
in response to the first similarity being not less than the preset similarity, updating the preset parameter by using the reference preset parameter and performing the eight-neighborhood search on the stimulation contact based on the updated preset parameter to acquire the isolation contacts.

In some possible implementations, the therapy information includes a preset parameter corresponding to each stimulation contact. The preset parameter indicates a distance relationship or a position relationship between the stimulation contact and an isolation contact corresponding to the stimulation contact.

In some possible implementations, acquiring, for each stimulation contact, multiple contacts as isolation contacts, where the multiple contacts satisfy at least one of a preset distance from the stimulation contact or a preset position relationship with the stimulation contact, includes:
inputting a stimulation parameter corresponding to the stimulation contact into a prediction model to acquire a reference preset parameter corresponding to the stimulation contact;
acquiring a first similarity between the reference preset parameter and the preset parameter;
in response to the first similarity being less than a preset similarity, performing an eight-neighborhood search on the stimulation contact based on the preset parameter to acquire the isolation contacts; and
in response to the first similarity being not less than the preset similarity, updating the preset parameter by using the reference preset parameter and performing the eight-neighborhood search on the stimulation contact based on the updated preset parameter to acquire the isolation contacts.

In some possible implementations, the control method also includes:
in response to the first similarity being less than the preset similarity, adding one to a count calculated by the counting module and determining whether the count added by one is greater than a preset count; and
in response to the count added by one being greater than the preset count, generating risk prompt information and clearing the count added by one.

In some possible implementations, the control method also includes:
determining the score of each stimulation contact according to a stimulation parameter and collection information corresponding to each stimulation contact and using the stimulation contact with the highest score as a recommended stimulation contact.

In a fourth aspect, the present application provides an electronic device. The electronic device includes a memory and a processor. The memory is configured to store a computer program. The processor is configured to, when executing the computer program, perform any preceding control method of the stimulator.

In a fifth aspect, the present application provides a computer-readable storage medium storing a computer program which, when executed by at least one processor, causes the at least one processor to perform any preceding control method of the stimulator.

In a sixth aspect, the present application provides a computer program product. The computer program product includes a computer program. The computer program is configured to, when executed by at least one processor, cause the at least one processor to perform any preceding method.

### BRIEF DESCRIPTION OF DRAWINGS

FIG. 1 is a block diagram of an implantable electrode according to embodiments of the present application.
FIG. 2 is a partial section view of a switch module according to embodiments of the present application.
FIG. 3 is a diagram illustrating the structure of an implantable electrode according to embodiments of the present application.
FIG. 4 is a diagram illustrating the positions of the contacts of an electrode array module according to embodiments of the present application.
FIG. 5 is a flowchart of a control method of a stimulator according to embodiments of the present application.
FIG. 6 is a flowchart of isolation contact acquisition according to embodiments of the present application.
FIG. 7 is a flowchart of a control method of a stimulator according to embodiments of the present application.
FIG. 8A is a block diagram of a stimulator according to embodiments of the present application.
FIG. 8B is a block diagram of a stimulator according to embodiments of the present application.
FIG. 9 is a block diagram of an electronic device according to embodiments of the present application.
FIG. 10 is a diagram illustrating the structure of a computer program product according to embodiments of the present application.

### DETAILED DESCRIPTION

The technical solutions in the present application are described below in conjunction with the drawings and the embodiments of the present application. Under the premise of no conflict, the embodiments or the technical features described below may be arbitrarily combined to form a new embodiment.

In the embodiments of the present application, words such as "exemplary" or "for example" are used for indicating examples, illustrations, or descriptions. Any embodiment or design solution that is described as "exemplary" or "for example" in the embodiments of the present application should not be construed as being more preferred or advantageous than other embodiments or design solutions. Specifically, the use of words such as "exemplary" or "for example" is intended to present relevant concepts in a certain manner.

The terms such as "first" and "second" in embodiments of the present application are used to illustrate and distinguish between the described objects. They do not imply any order, nor do they indicate any special limitation on quantity in embodiments of the present application. These terms do not impose any limitation on embodiments of the present application.

Hereinafter, one application field of embodiments of the present application (that is, an implantable medical device) is briefly described.

An implantable medical system includes an implantable neural electrical stimulation system, an implantable cardiac electrical stimulation system (also referred to as a cardiac pacemaker), an implantable drug delivery system (IDDS), and a lead adapter system. The implantable neural electrical stimulation system is, for example, a deep brain stimulation (DBS) system, an implantable cortical nerve stimulation (CNS) system, an implantable spinal cord stimulation (SCS) system, an implantable sacral nerve stimulation (SNS) system, or an implantable vagus nerve stimulation (VNS) system.

An implantable neural electrical stimulation system includes a stimulator implanted in a patient (that is, an implantable neural stimulator) and a program-controlled device disposed outside the patient. That is, a stimulator is a medical device, or, in other words, a medical device includes a stimulator. The related neuromodulation technology mainly involves implanting electrodes (for example, in the form of electrode leads) at specific sites (that is, targets) of tissue in a biological organism via stereotactic surgery, delivering electrical pulses to the targets through the electrodes and modulating the electrical activity and function of the corresponding neural structures and networks, thereby improving symptoms and alleviating pain.

In an example, the DBS system includes an implantable pulse generator (IPG), an extension lead, and an electrode, with the IPG connected to the electrode via the extension lead. The IPG is implanted in the patient, for example, in the chest or other internal locations of the patient.

In another example, the DBS system includes an IPG and an electrode, with the IPG directly connected to the electrode. The IPG is implanted in the patient's head, for example, by creating a groove in the patient's skull and then placing the IPG into the groove. In this case, the IPG may be fully recessed within the skull or may partially protrude from the outer surface of the skull.

The IPG, in response to programming instructions sent by a program-controlled device, delivers controllable electrical stimulation therapy (or electrical stimulation energy) to the tissue of the patient by using a sealed battery and circuitry. When the battery level is low, the battery needs to be recharged, and the charging method may involve wireless charging using an electromagnetic induction coil, with the charging occurring through the patient's skin or other epidermal tissue. The IPG delivers one or multiple controllable specific electrical stimulations to specific regions of the tissue of the patient through the electrode.

In some embodiments, the extension lead is used in conjunction with the IPG as a medium for transmitting electrical stimulation, conveying the electrical stimulation generated by the IPG to the electrode.

In some embodiments, the electrical stimulation may be delivered in the form of a pulsed signal or in the form of a non-pulsed signal. For example, the electrical stimulation may be delivered as a signal having various waveform shapes, frequencies, and amplitudes. Therefore, electrical stimulation in the form of a non-pulsed signal may be a continuous signal that may have a sinusoidal waveform or other continuous waveforms.

Embodiments of the present application are not limited to any particular disease type and can be applicable to disease types suitable for DBS, SCS, sacral nerve stimulation, gastric stimulation, peripheral nerve stimulation, or functional electrical stimulation. Disease types that may be treated or managed by the DBS include, but are not limited to, seizure disorders (such as epilepsy), pain, migraine, mental illnesses (such as major depressive disorder (MDD)), bipolar disorder, anxiety disorder, post-traumatic stress disorder, dysthymia, obsessive-compulsive disorder (OCD), behavior disorder, mood disorder, memory disorder, mental state disorder, movement disorders (such as essential tremor or Parkinson's disease), Huntington's disease, Alzheimer's disease, drug addiction, autism or other neurological or psychiatric disorders and impairment.

In embodiments of the present application, when a programming connection is established between the program-controlled device and the stimulator, the program-controlled device can be used to adjust one or more stimulation parameters of the stimulator (or one or more stimulation parameters of the pulse generator, where different stimulation parameters correspond to different electrical stimulations). The stimulator can also collect the patient's electrophysiological activity to acquire electrophysiological signals, and the acquired electrophysiological signals can be used to further adjust the stimulator's stimulation parameters, thereby achieving closed-loop control (or adaptive adjustment) of the stimulation parameters.

The stimulation parameters may include at least one of the following: a stimulation contact identifier (for example, a 2# electrode contact or a 3# electrode contact) for delivering electrical stimulation, a frequency (for example, the number of electrical stimulation pulse signals per 1s, in units of Hz), a pulse width (the duration of each pulse, in units of µs), an amplitude (generally represented by voltage, that is, the strength of each pulse, in units of V), a timing (for example, continuation or burst, where burst refers to a discontinuous timing behavior formed by multiple processes), a stimulation mode (including one or more of a current mode, a voltage mode, a timing stimulation mode, and a cyclic stimulation mode), physician-controlled upper and lower limits (a range that can be adjusted by a physician), or patient-controlled upper and lower limits (a range that can be autonomously adjusted by the patient).

In some embodiments, at least one stimulation parameter of the stimulator may be adjusted in the current mode or the voltage mode.

The program-controlled device may include a physician program-controlled device (that is, a program-controlled device used by a physician) and/or a patient program-controlled device (that is, a program-controlled device used by a patient). The physician program-controlled device may be, for example, a tablet computer, a laptop, a desktop computer, a smartphone, or another smart terminal device equipped with programming software. The patient program-controlled device may be, for example, a tablet computer, laptop, desktop computer, smartphone, or another smart terminal device equipped with programming software. The patient program-controlled device may also be other electronic devices with programming functions (for example, a charger or an electrophysiological signal acquisition device with programming capabilities).

The data interaction between the physician program-controlled device and the stimulator is not limited in embodiments of the present application. When the physician performs remote programming, the physician program-controlled device can perform data interaction with the stimulator via a server and the patient program-controlled device. When the physician performs offline programming with the patient face to face, the physician program-controlled device may perform data interaction with the stimulator via the patient program-controlled device or directly.

In some embodiments, the patient program-controlled device may include a host (in communication with the server) and a slave (in communication with the stimulator) that are communicatively connected to each other. The physician program-controlled device may exchange data with the server via 3rd-generation/4th-generation/5th-generation mobile communication technologies (3G/4G/5G). The server may exchange data with the host via the 3G/4G/5G network. The host may exchange data with the slave via the Bluetooth/Wireless Fidelity (Wi-Fi)/Universal Serial Bus (USB) protocol. The slave may exchange data with the stimulator via the 401 MHz-406 MHz or 2.4 GHz-2.48 GHz frequency bands. The physician program-controlled device may also directly exchange data with the stimulator via the 401 MHz-406 MHz or 2.4 GHz-2.48 GHz frequency bands.

In the related art, considering the use of electrode contacts to deliver stimulation and collect signals, to achieve closed-loop control of the stimulator, the collected signal is mainly processed as follows:
Filtering is performed. A filter is used to select signals within a specific frequency range to remove noise at other frequencies.

Denoising algorithms such as wavelet transform and independent component analysis (ICA) are used to separate useful signal components from noise components.

However, the preceding processing methods do not address the problem at its source, that is, during the signal collection process. Based on this, the present application provides an implantable electrode, a stimulator and a control method therefor, and a computer-readable storage medium. Electrical stimulation and physiological signal collection are achieved in the tissue of a patient by using a control module, an array switch, and an electrode array module. Under the control of a switch control signal, isolation is established between electrical stimulation and signal collection, thereby reducing interference from electrical stimulation on the collection at the source and improving the accuracy of closed-loop control of the stimulator.

The following describes the implantable electrode for the stimulator, the control method of the stimulator, and the stimulator.

### Embodiment of the implantable electrode

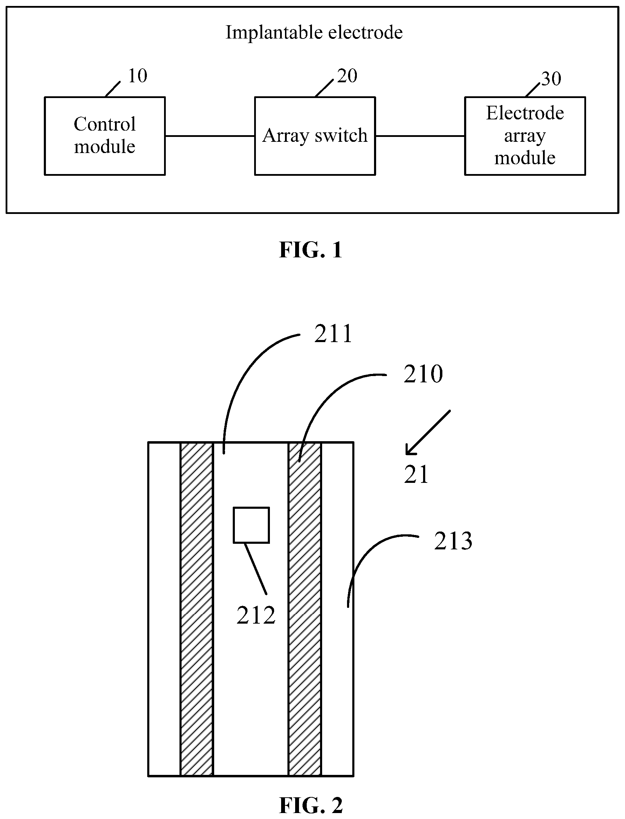

Referring to FIG. 1 to FIG. 4, FIG. 1 is a block diagram of an implantable electrode according to embodiments of the present application.

This embodiment provides an implantable electrode. The implantable electrode includes a control module 10, an array switch 20, and an electrode array module 30.

The control module 10 is configured to receive a gating instruction from a pulse generator and generate a switch control signal.

The array switch 20 is configured to establish multiple gated channels according to the switch control signal.

The electrode array module 30 includes multiple contacts 31. Each contact 31 is configured as a stimulation contact to deliver electrical stimulation to tissue of a patient through a gated channel established by the array switch 20 or configured as a collection contact to collect a physiological signal from tissue of a patient through a gated channel established by the array switch 20.

The switch control signal is used for isolating the stimulation contact for delivering the electrical stimulation from the collection contact for collecting the physiological signal.

Thus, under the control of the switch control signal, physical isolation is established between electrical stimulation and signal collection, reducing interference from electrical stimulation on the collected signal and improving the accuracy of the acquired data. Selective activation of specific electrode contacts allows targeting and treating particular stimulation regions, thereby improving therapeutic efficacy. The electrode array module (equivalent to the preceding electrode) is configured not only to perform electrical stimulation but also to collect a physiological signal from the patient. This enables a user to monitor the patient's physiological state in real time and make necessary adjustments. In summary, electrical stimulation and physiological signal collection are achieved simultaneously in the tissue of the patient by using the control module, the array switch, and the electrode array module. In addition, interference from electrical stimulation on the collected signal is reduced.

Physiological signals may include neuronal signals and/or local field potential signals. Neuronal signals are electrical signals generated by neurons and used for information transmission between neurons. Neuronal signals usually exist in the form of pulses, referred to as action potentials. The collection and analysis of neuronal signals can provide information about neuronal activity, thereby helping to understand the function and abnormalities of the nervous system.

Local field potential signals refer to potential changes generated by the electrical activity of surrounding neurons. The collection of local field potential signals can provide information about neural network activity and the behavior of neuronal populations. An analysis of local field potential signals can provide insights into the synchrony of neuronal populations, regulatory mechanisms, and electrical activities related to specific functions. The collection and analysis of neuronal signals and/or local field potential signals can provide important information about nervous system function and disease states, helping to diagnose and treat neurological disorders and enabling a personalized closed-loop deep brain stimulation therapy.

In some embodiments, the switch control signal includes a high-level signal and a low-level signal, the high-level signal is used for gating a channel of the array switch 20, and the low-level signal is used for disconnecting a channel of the array switch 20.

High-level and low-level signals are used to improve the accuracy of controlling the channel states of the array switch so that connection is established when required and disconnection is achieved when not required, meeting the requirements for therapy or signal collection. A combination of high-level and low-level signals enables diverse bidirectional communication for real-time closed-loop control and monitoring.

High-level indicates that the electrical signal is at a relatively high voltage level. Low-level indicates that the electrical signal is at a relatively low voltage level.

In an example, 3.3 volts is used as a high level, and 0 volts is used as a low level.

In another example, a voltage between 1.65 volts and 5 volts is considered a high level, and a voltage below 1.65 volts is considered a low level.

Channel disconnection means that the connection in the circuit is opened or interrupted, preventing current from flowing through the connection. Channel gated means that the connection in the circuit is closed or connected, allowing current to flow freely through the connection.

Referring to FIG. 2, FIG. 2 is a partial section view of a switch module according to embodiments of the present application.

In some embodiments, the array switch 20 includes multiple switch modules 21.

Each switch module 21 includes an insulating layer 210, a switch unit 212, and a conductive layer 213.

The insulating layer 210 is configured to form an accommodation cavity 211.

The switch unit 212 is disposed in the accommodation cavity 211 formed by the insulating layer 210 and configured to establish, according to the switch control signal, a gated channel of a contact 31 corresponding to the switch control signal.

The conductive layer 213 coats the outer surface of the insulating layer 210 and is configured to be grounded through a wire to shield at least one of electric-field interference or electromagnetic interference.

Each switch module 21 includes an insulating layer 210 configured to form an accommodation cavity 211. The switch unit 212 is disposed in the accommodation cavity 211 formed by the insulating layer 210 and is configured to establish a gated channel for the contact 31 corresponding to the switch control signal based on the switch control signal. This can be understood as that, by controlling the switch unit 212, it is possible to selectively connect or disconnect the channel between the contacts 31. The conductive layer 213 coats the outer surface of the insulating layer 210 and is configured to be grounded through a wire to shield at least one of electric-field interference or electromagnetic interference. It can be considered that the conductive layer 213 helps reduce the impact of external interference on the collected signal passing through the switch unit 212.

The insulating layer 210 may be composed of one or more of a ceramic material, a resin material, and a polymer material to achieve insulation between the switch unit 212 in the accommodation cavity 211 and the exterior of the insulating layer 210 (for example, the conductive layer). The conductive layer 213 may be made of a metal material such as copper or silver or may be made of a carbon-based conductive material such as graphite used for the fabrication of a flexible printed circuit board. The switch unit 212 is one of the basic constituent units of the switch module 21 of the array switch 20. The switch unit 212 is located in the insulating layer 210 and is configured to enable electrical connection between the contacts 31 or isolate the contacts 31 from each other. The switch unit 212 may take various forms and structures, such as an electronic switch, a magnetic switch, an electrochemical switch, or an optical switch.

It is generally understood that signals passing through the switch unit 212 are negative electrons. The conductive layer 213 is grounded so that any charge or magnetic field that passes through the current switch module and comes into contact with the conductive layer 213 is directed outward (to the grounding point connected by a wire), thereby shielding against electric and electromagnetic interference. Moreover, the shielding layer can be used to block or weaken external electromagnetic or electric field interference on the interior, and the combination of the conductive layer 213 and the shielding layer provides effective shielding and isolation.

In summary, by establishing physical isolation between electrical stimulation and signal collection under the control of the switch control signal, it is possible to reduce interference from electrical stimulation on the collected signal, thereby better shielding against electric and electromagnetic interference and improving the accuracy and stability of signal collection.

In application, an upper connection portion is disposed between each switch module 21 and the control module 10, and a lower connection portion is disposed between each switch module 21 and the contact 31 of the electrode array module 30. The upper connection portion is configured to establish an electrical connection between the switch module 21 and the control module 10. The lower connection portion is configured to establish an electrical connection between the switch module 21 and the contact 31. In some embodiments, the electrode array module 30 is a Utah electrode, and/or the physiological signal is a local field potential signal.

The Utah electrode may include multiple electrode contacts. The contact 31 can be configured for physiological signal collection and electrical stimulation simultaneously. This multi-channel functionality allows for the collection or stimulation of different neural regions at the same time, providing more therapeutic options. The multi-electrode layout of the Utah electrode provides high spatial resolution for signal collection and stimulation, thereby enabling precise targeting and treatment of specific neural regions. The Utah electrode is miniature and flexible and can be implanted into the patient through minimally invasive surgery, thereby reducing surgical trauma and recovery time. Moreover, the local field potential signal primarily reflects a physiological activity in a specific region, enabling the collected signal to represent the state of the tissue more accurately.

Because the local field potential signal encompasses the activity of a large number of neurons and thus provides more comprehensive information, offering insights into the overall state of the nervous system while reducing the need for complex measurements at the level of individual neurons.

The body of the Utah electrode may be made of a silicon wafer. The tip of the needle-shaped contact of the Utah electrode may be coated with platinum or other highly conductive metals for delivering electrical stimulation to the tissue or collecting a physiological electrical signal from the tissue. Moreover, portions of the contact other than the tip may be insulated through materials such as polyimide.

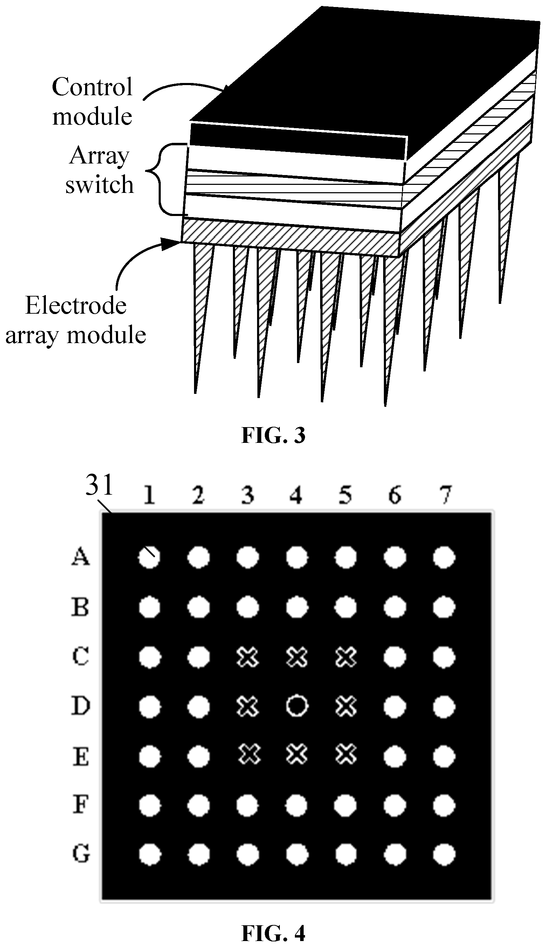

Referring to FIG. 3, FIG. 3 is a diagram illustrating the structure of an implantable electrode according to embodiments of the present application.

In an application scenario, this embodiment of the present application provides an implantable electrode. The implantable electrode includes a control module, an array switch, and an electrode array module.

The control module is configured to receive a gating instruction from a pulse generator and generate a switch control signal. The switch control signal includes a high-level signal and a low-level signal. The high-level signal is used for gating a channel of the array switch 20. The low-level signal is used for disconnecting a channel of the array switch 20 to isolate the stimulation contact from the collection contact.

The array switch is configured to establish multiple gated channels according to the switch control signal. The electrode array module is a Utah electrode.

The electrode array module includes multiple contacts. Each contact is configured as a stimulation contact to deliver electrical stimulation to tissue of a patient through a gated channel established by the array switch or configured as a collection contact to collect a physiological signal from tissue of a patient through a gated channel established by the array switch. The physiological signal is a local field potential signal.

The array switch can establish or break connections by using use various mechanisms. This is not limited in the present application. For example, the array switch may include multiple electronic switches, magnetic switches, electrochemical switches, or optical switches. An electronic switch can use a solid-state electronic component (such as a transistor or a relay) to control the opening or closing of a channel, enabling very fast switching of the channel state. A magnetic switch can use a magnetic field to control the opening or closing of a channel. In general, when the magnetic field is adjusted, the magnetic switch changes the channel state. An electrochemical switch can use an electrochemical reaction to control the opening or closing of a channel. An electrochemical switch contains an electrolyte material and can change the channel state by controlling the electrochemical reaction. An optical switch can use an optical signal to control the channel state. For example, an optical switch can use optical fibers or optical components to allow or prevent the transmission of a light signal.

In an example, referring to FIG. 4, FIG. 4 is a diagram illustrating the positions of the contacts of an electrode array module according to embodiments of the present application.

The contacts 31 in the electrode array module are marked as follows: The electrodes marked with "×" (for example, C3, C4, C5, D3, D5, E3, E4, E5) represent the contacts 31 corresponding to channels that are turned off under the control of the low-level signal. The dot (D4) located at the center of the electrode region marked with "×" can serve as a stimulation contact configured to deliver electrical stimulation to the tissue of the patient through a gated channel established by the array switch under the control of the high-level signal. The dots (for example, A1, A2) located around the electrode region marked with "×" can serve as collection contacts to collect physiological signals from the tissue of the patient. The high-level signal is used for gating a channel of the array switch 20. The low-level signal is used for disconnecting a channel of the array switch 20 to isolate the stimulation contact from the collection contact.

### Method embodiment

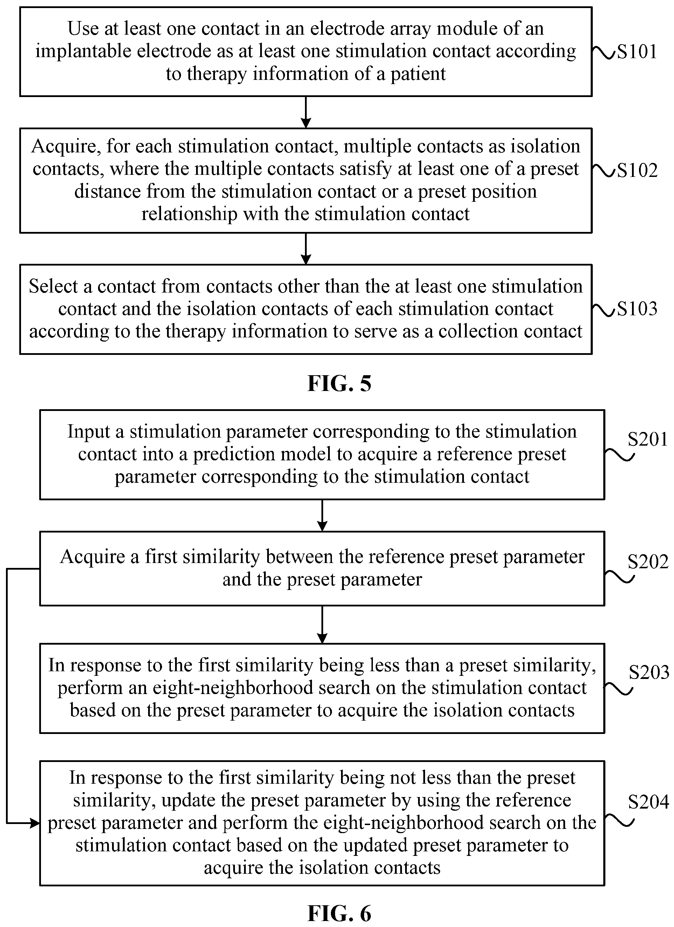

Referring to FIG. 5, FIG. 5 is a flowchart of a control method of a stimulator according to embodiments of the present application.

In this method embodiment, the stimulator is configured to be implanted into a patient and configured to deliver electrical stimulation to and/or collect a physiological signal from the tissue of the patient. The stimulator includes the implantable electrode of the embodiment of the electrode and a pulse generator. The implantable electrode is consistent with the previous embodiment of the electrode in terms of technical effects, and thus some content is not described here.

The control method of the stimulator includes the following steps:
In step S101, at least one contact in an electrode array module of an implantable electrode is used as at least one stimulation contact according to therapy information of a patient, where each stimulation contact is configured to deliver electrical stimulation to the tissue of the patient.

In step S102, for each stimulation contact, multiple contacts as isolation contacts are acquired, where the multiple contacts satisfy at least one of a preset distance from the stimulation contact or a preset position relationship with the stimulation contact.

In step S103, at least one contact is selected from contacts other than the at least one stimulation contact and the isolation contacts of each stimulation contact according to the therapy information to serve as at least one collection contact; and, when the stimulation contact delivers the electrical stimulation to the tissue of the patient, a physiological signal is collected from the tissue of the patient by using the collection contact, and the collected physiological signal is used as collection information.

Thus, through the cooperation between the pulse generator and the implantable electrode, the electrical stimulation parameters can be adjusted based on the patient's physiological signal as feedback information to meet the patient's specific needs (that is, personalized needs) and to achieve closed-loop control of the electrical stimulation delivered to the patient. Isolation contact selection can reduce unnecessary effects of the stimulation on surrounding tissue, thereby lowering treatment risks and side effects. By determining the stimulation contacts, isolation contacts, and collection contacts in the preceding manner and then using them for treatment execution - where the stimulation contacts are configured to deliver electrical stimulation to the tissue and the collection contacts are configured to monitor the patient's physiological response - an optimal monitoring effect can be achieved.

For example, the therapy information may include the patient's condition, such as essential tremor, Parkinson's disease, or other disorders. Different conditions correspond to different target nuclei for treatment. Based on the therapy information, one or more contacts in the electrode array module of the implantable electrode that are closest to the target nucleus may be selected as stimulation contacts. Based on the therapy information, contacts are selected from those other than the stimulation contact and isolation contacts to serve as collection contacts. This may be understood as selecting the contacts closest to the stimulation contact among those not designated as stimulation and isolation contacts so as to obtain objective feedback on the patient's response to the electrical stimulation. The therapy information may also include the positions of one or more target stimulation contacts and the stimulation parameters corresponding to each of these contacts.

Referring to FIG. 6, FIG. 6 is a flowchart of isolation contact acquisition according to embodiments of the present application.

In some embodiments, the therapy information includes a preset parameter corresponding to each stimulation contact. The preset parameter indicates a distance relationship or a position relationship between the stimulation contact and an isolation contact corresponding to the stimulation contact.

Acquiring, for each stimulation contact, multiple contacts as isolation contacts by an eight-neighborhood search, where the multiple contacts satisfy at least one of a preset distance from the stimulation contact or a preset position relationship with the stimulation contact, includes the following steps:
In step S201, a stimulation parameter corresponding to the stimulation contact is input into a prediction model to acquire a reference preset parameter corresponding to the stimulation contact.

In step S202, a first similarity between the reference preset parameter and the preset parameter is acquired.

In step S203, in response to the first similarity being less than a preset similarity, an eight-neighborhood search is performed on the stimulation contact based on the preset parameter to acquire the isolation contacts.

In step S204, in response to the first similarity being not less than the preset similarity, the preset parameter is updated by using the reference preset parameter, and the eight-neighborhood search is performed on the stimulation contact based on the updated preset parameter to acquire the isolation contacts.

Thus, the eight-neighborhood search enables errors in the contact selection process to be reduced, thereby improving the accuracy and reliability of the treatment. If the first similarity is less than the preset similarity, this indicates that there is a significant difference between the preset parameter of the stimulation contact and the reference preset parameter. The preset parameter is determined by the user based on earlier calibration and testing and thus has relatively high stability and reliability. In this case, the preset parameter of the stimulation contact is used for eight-neighborhood search to acquire the isolation contacts. If the first similarity is not less than the preset similarity, this indicates that the preset parameter of the stimulation contact is relatively close to the reference preset parameter. The preset parameter is then updated, and the updated preset parameter is used for eight-neighborhood search to acquire the isolation contacts.

Eight-neighborhood search is an algorithm used for identifying points or elements adjacent to a central point. The search process includes determining a central point and identifying eight neighboring points around the central point. The neighboring points may be located above, below, to the left, to the right, or along the four diagonal directions of the central point. Since the contacts on the electrode array module are typically arranged in a regular pattern, the eight-neighborhood search is more efficient. In application, other related heuristic algorithms (neighborhood search algorithms) based on "neighborhood" may also be used.

The first similarity between the reference preset parameter and the preset parameter may be obtained by using metrics such as Euclidean distance, Manhattan distance, or cosine similarity to quantify the difference between the two sets of parameters into a single value. A smaller value indicates higher similarity. The first similarity between the reference preset parameter and the preset parameter may also be obtained by calculating the correlation coefficient between the two sets of parameters, such as the Pearson correlation coefficient or the Spearman rank correlation coefficient. The correlation coefficient represents the degree of linear or nonlinear correlation between the two sets of parameters. When the value of the correlation coefficient is close to 1, the similarity is high.

The prediction model may be trained based on a machine learning algorithm by using training data consisting of stimulation parameters corresponding to the known stimulation contacts and their corresponding reference preset parameters. For the trained prediction model, upon receiving the stimulation parameters, the prediction model outputs the corresponding predicted preset parameters and use these parameters as the reference preset parameters. The prediction model may be obtained through training or may be a pre-trained prediction model.

In some embodiments, the training process of the prediction model includes acquiring a training set. The training set includes multiple training data items. Each training data item includes a stimulation parameter and labeled data of the preset parameter corresponding to the stimulation parameter.

For each training data item in the training set, the training process includes:
inputting the stimulation parameter from the training data item into a preset deep learning model to obtain the prediction data of the preset parameter corresponding to the stimulation parameter;
updating the model parameters of the deep learning model based on the prediction data and the labeled data of the preset parameter corresponding to the stimulation parameter; and
determining whether a preset training termination condition is satisfied; if yes, using the trained deep learning model as the prediction model; if not, continuing training the deep learning model by using the next training data item.

In this manner, an appropriate number of neuron computing nodes and a multi-layer computational hierarchy are established through a design, and an appropriate input layer and an appropriate output layer are selected to obtain the preset first deep learning model. A functional relationship between input and output is established through the learning and optimization of the preset first deep learning model. Although the functional relationship between input and output cannot be 100% found, a realistic correlation can be approached as close as possible. The prediction model trained in this manner can obtain corresponding output data based on input data, has a wide range of applicability, and provides high accuracy and reliability in its computational results.

In some embodiments, the method also includes:
in response to the first similarity being less than the preset similarity, adding one to the count calculated by the counting module and determining whether the count added by one is greater than a preset count; and
in response to the count added by one being greater than the preset count, generating risk prompt information and clearing the count added by one.

If the first similarity is less than the preset similarity, this indicates that there is a considerable difference between the preset parameter of the stimulation contact and the reference preset parameter. In the case where the first similarity is less than the preset similarity, the number of occurrences of the similarity difference is recorded, and it is determined whether the recorded count exceeds the preset count. When the recorded count exceeds the preset count, risk prompt information is generated to alert the user or prompt a further action.

In this embodiment, no limitation is imposed on the preset similarity. The preset similarity may be, for example, 0.81, 0.83, 65%, 75%, 87%, 88%, 89%, or 95%.

The risk prompt information may be sent to the user device in the form of voice, image, or text. The user device may be, for example, a tablet, mobile phone, or program-controlled device used by a physician or the patient's caregiver. In an example, when the count reaches 10, exceeding the preset count of 9, the risk prompt information is sent via voice to the mobile phone of Dr. B, the attending physician of patient A. The phone automatically plays the voice message, "the stimulation treatment status of patient A requires your attention", enabling the physician to be immediately informed of the risk and take notice or further action.

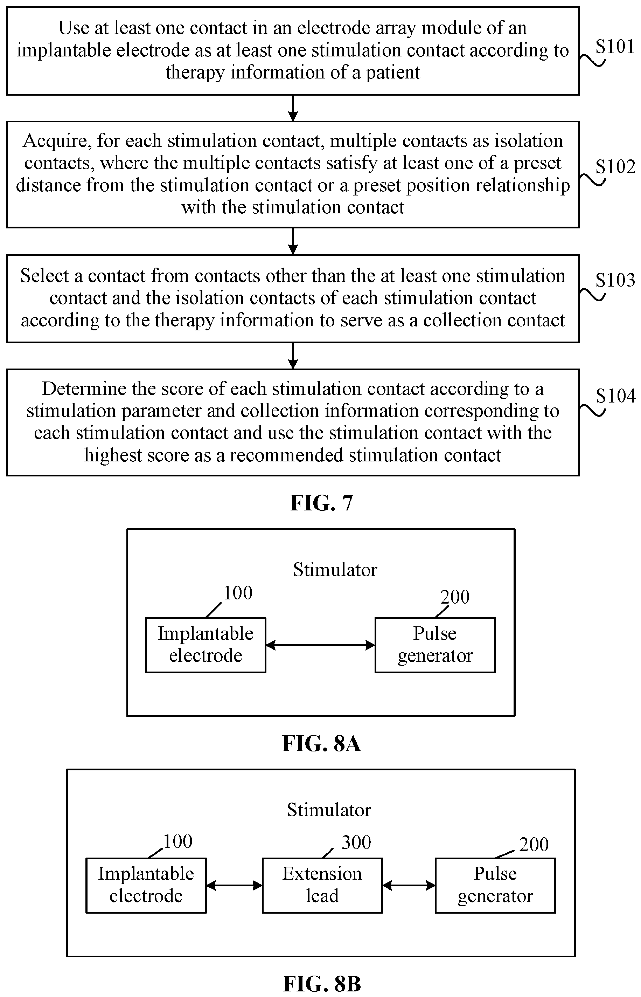

Referring to FIG. 7, FIG. 7 is a flowchart of a control method of a stimulator according to embodiments of the present application.

In some embodiments, the method also includes the following:
In step S104, the score of each stimulation contact is determined according to a stimulation parameter and collection information corresponding to each stimulation contact, and the stimulation contact with the highest score is used as a recommended stimulation contact.

Thus, by scoring and selecting the stimulation contact with the highest score, the user can be assisted in making treatment decisions more efficiently.

The physiological response of each stimulation contact can be used as the basis for scoring. For example, the amplitude, frequency, or phase of the physiological signal generated by the stimulation contact can be used to assess the effect of stimulation, with larger physiological responses receiving higher scores.

In an application scenario, embodiments of the present application also provide a control method of a stimulator. The method includes:
using at least one contact in an electrode array module of an implantable electrode as at least one stimulation contact according to therapy information of a patient, where each stimulation contact is configured to deliver electrical stimulation to tissue of the patient;
for each stimulation contact, inputting a stimulation parameter corresponding to the stimulation contact into a prediction model to acquire a reference preset parameter corresponding to the stimulation contact;
acquiring a first similarity between the reference preset parameter and the preset parameter; and
in response to the first similarity being not less than the preset similarity, updating the preset parameter by using the reference preset parameter and performing the eight-neighborhood search on the stimulation contact based on the updated preset parameter to acquire the isolation contacts.

When the first similarity is less than a preset similarity, an eight-neighborhood search is performed on the stimulation contact based on the preset parameter to acquire the isolation contacts; and one is added to a count calculated by the counting module, and it is determined whether the count added by one is greater than a preset count. When the count added by one is greater than the preset count, risk prompt information is generated, and the count added by one is cleared.

At least one contact is selected from contacts other than the at least one stimulation contact and the isolation contacts of each stimulation contact according to the therapy information to serve as at least one collection contact. When the stimulation contact delivers the electrical stimulation to the tissue of the patient, a physiological signal is collected from the tissue of the patient by using the collection contact, and the collected physiological signal is used as collection information.

The score of each stimulation contact is determined according to a stimulation parameter and collection information corresponding to each stimulation contact, and the stimulation contact with the highest score is used as a recommended stimulation contact.

### Embodiment of the stimulator

Referring to FIG. 8A and FIG. 8B, FIG. 8A is a block diagram of a stimulator according to embodiments of the present application, and FIG. 8B is a block diagram of another stimulator according to embodiments of the present application. The two stimulators have different structures.

The stimulator includes any preceding implantable electrode 100 and a pulse generator.

The implantable electrode 100 is configured to deliver the electrical stimulation to the tissue of the patient and/or collect the physiological signal from the tissue of the patient.

The (implantable) pulse generator 200 is electrically connected to the implantable electrode 100 directly or through an extension lead 300 and configured to parse the physiological signal and generate the electrical stimulation.

The implantable electrode 100 is consistent with the previous embodiment of the electrode in terms of technical effects, and thus some content is not described here.

Thus, through the cooperation between the pulse generator 200 and the implantable electrode 100, the electrical stimulation parameters can be adjusted based on the patient's physiological signal as feedback information to meet the patient's specific needs and conditions and to achieve closed-loop control of the electrical stimulation delivered to the patient.

In some embodiments, the pulse generator 200 also includes a stimulation contact determination module, an isolation contact acquisition module, and a collection contact determination module.

The stimulation contact determination module is configured to use at least one contact in the electrode array module as at least one stimulation contact according to therapy information of the patient. Each stimulation contact is configured to deliver the electrical stimulation to the tissue of the patient.

The isolation contact acquisition module is configured to acquire, for each stimulation contact, multiple contacts as isolation contacts. The multiple contacts satisfy at least one of a preset distance from the stimulation contact or a preset position relationship with the stimulation contact.

The collection contact determination module is configured to select a contact from contacts other than the at least one stimulation contact and the isolation contacts of each stimulation contact according to the therapy information to serve as a collection contact; and, when the stimulation contact delivers the electrical stimulation to the tissue of the patient, collect the physiological signal from the tissue of the patient by using the collection contact and take the collected physiological signal as collection information.

Thus, isolation contact selection can reduce unnecessary effects of the stimulation on surrounding tissue, thereby lowering treatment risks and side effects. By determining the stimulation contacts, isolation contacts, and collection contacts in the preceding manner and then using them for treatment execution - where the stimulation contacts are configured to deliver electrical stimulation to the tissue and the collection contacts are configured to monitor the patient's physiological response - an optimal monitoring effect can be achieved.

In some embodiments, the therapy information includes a preset parameter corresponding to each stimulation contact. The preset parameter indicates a distance relationship or a position relationship between the stimulation contact and an isolation contact corresponding to the stimulation contact.

In some embodiments, the isolation contact acquisition module includes a parameter prediction unit, a similarity determination unit, a first contact acquisition unit, and a second contact acquisition unit.

The parameter prediction unit is configured to input a stimulation parameter corresponding to the stimulation contact into a prediction model to acquire a reference preset parameter corresponding to the stimulation contact.

The similarity determination unit is configured to acquire a first similarity between the reference preset parameter and the preset parameter.

The first contact acquisition unit is configured to, in response to the first similarity being less than a preset similarity, perform an eight-neighborhood search on the stimulation contact based on the preset parameter to acquire the isolation contacts.

The second contact acquisition unit is configured to, in response to the first similarity being not less than the preset similarity, update the preset parameter by using the reference preset parameter and perform the eight-neighborhood search on the stimulation contact based on the updated preset parameter to acquire the isolation contacts.

Thus, the eight-neighborhood search enables errors in the contact selection process to be reduced, thereby improving the accuracy and reliability of the treatment. If the first similarity is less than the preset similarity, this indicates that there is a significant difference between the preset parameter of the stimulation contact and the reference preset parameter. The preset parameter is determined by the user based on earlier calibration and testing and thus has relatively high stability and reliability. In this case, the preset parameter of the stimulation contact is used for eight-neighborhood search to acquire the isolation contacts. If the first similarity is not less than the preset similarity, this indicates that the preset parameter of the stimulation contact is relatively close to the reference preset parameter. The preset parameter is then updated, and the updated preset parameter is used for eight-neighborhood search to acquire the isolation contacts.

In some embodiments, the pulse generator 200 also includes a counting module and a risk prompt module.

The counting module is configured to, in response to the first similarity being less than the preset similarity, add one to a count calculated by the counting module and determine whether the count added by one is greater than a preset count.

The risk prompt module is configured to, in response to the count added by one being greater than the preset count, generate risk prompt information and clear the count added by one.

If the first similarity is less than the preset similarity, this indicates that there is a considerable difference between the preset parameter of the stimulation contact and the reference preset parameter. In the case where the first similarity is less than the preset similarity, the number of occurrences of the similarity difference is recorded, and it is determined whether the recorded count exceeds the preset count. When the recorded count exceeds the preset count, risk prompt information is generated to alert the user or prompt a further action.

In some embodiments, the pulse generator 200 also includes a recommended-contact acquisition module.

The recommended-contact acquisition module is configured to determine the score of each stimulation contact according to a stimulation parameter and collection information corresponding to each stimulation contact and use the stimulation contact with the highest score as a recommended stimulation contact.

Thus, by scoring and selecting the stimulation contact with the highest score, the user can be assisted in making treatment decisions more efficiently.

### Embodiment of the electronic device

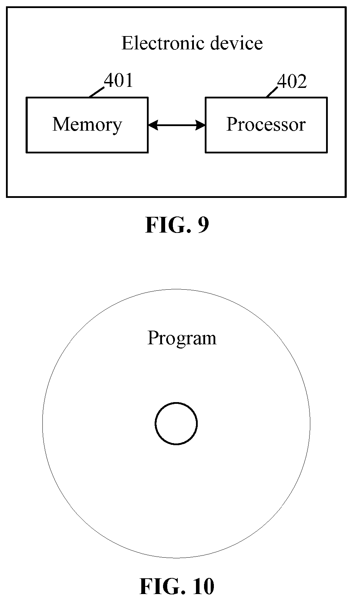

As shown in FIG. 9, this embodiment of the present application provides an electronic device. The electronic device includes a memory 401 and a processor 402. The memory 401 is configured to store a computer program. The processor 402 is configured to, when executing the computer program, perform any preceding method. The embodiment of the electronic device is consistent with the previous method embodiment in terms of technical effects, and thus some content is not described here.

### Embodiment of the storage medium

This embodiment of the present application provides a computer-readable storage medium. The embodiment of the storage medium is consistent with the previous method embodiment in terms of technical effects, and thus some content is not described here.

The computer-readable storage medium stores a computer program. The computer program is configured to, when executed by at least one processor, cause the at least one processor to perform the steps of any preceding method or the function of any preceding stimulator.

The computer-readable medium may be a computer-readable signal medium or a computer-readable storage medium. In the embodiments of the present application, the computer-readable storage medium may be any tangible medium including or storing a program. The program may be used by or in conjunction with an instruction execution system, apparatus, or device. The computer-readable storage medium may be, for example, an electronic, magnetic, optical, electromagnetic, infrared, or semiconductor system, apparatus, or device, or any combination thereof. Examples of the computer-readable storage medium include an electrical connection having one or more wires, a portable disk, a hard disk, a random-access memory (RAM), a read-only memory (ROM), an erasable programmable read-only memory (EPROM), a flash memory, an optical fiber, a portable compact disc read-only memory (CD-ROM), an optical storage device, a magnetic storage device, or any suitable combination thereof. The storage medium may be a non-transitory storage medium.

The computer-readable storage medium may include a data signal propagated in a baseband or as part of a carrier. Readable program codes are carried in the data signal. This propagated data signal may take multiple forms including an electromagnetic signal, an optical signal, or any suitable combination thereof. The computer-readable storage medium may also be any computer-readable medium, and the computer-readable medium may send, propagate or transmit a program used by or in combination with an instruction execution system, apparatus or device. The program codes included in the computer-readable storage medium may be transmitted using any appropriate medium, including wireless, wireline, fiber-optic cable, Radio Frequency (RF), or any appropriate combination thereof. The program codes for performing the operations of the present invention may be written in one programming language or any combination of multiple programming languages. The programming languages include object-oriented programming languages such as Java, C++ and further include conventional procedural programming languages such as C programming language or similar programming languages. The program codes may be executed entirely on a user computing device, executed partly on a user computing device, executed as a stand-alone software package, executed partly on a user computing device and partly on a remote computing device, or executed entirely on a remote computing device or a server. In the case where the remote computing device is involved, the remote computing device may be connected to the user computing device via any type of network including a local area network (LAN) or a wide area network (WAN) or may be connected to an external computing device (for example, via the Internet provided by an Internet service provider).

### Embodiment of the program product

This embodiment of the present application provides a computer program product. The embodiment of the program product is consistent with the previous method embodiment in terms of technical effects, and thus some content is not described here.

The computer program product includes a computer program. The computer program is configured to, when executed by at least one processor, cause the at least one processor to perform the steps of any preceding method or the function of any preceding stimulator.

Referring to FIG. 10, FIG. 10 is a diagram illustrating the structure of a computer program product according to embodiments of the present application.

The computer program product is configured to implement the steps of any preceding method. The program product may use a portable compact disk read-only memory (CD-ROM), may include a program code and may run on terminal devices such as a personal computer. However, the computer program product of the present application is not limited to this and may be any combination of one or more computer-readable media.

## Claims

1. An implantable electrode, comprising:
- a control module configured to receive a gating instruction from a pulse generator and generate a switch control signal;
- an array switch configured to establish a plurality of gated channels according to the switch control signal; and
- an electrode array module comprising a plurality of contacts, wherein each contact is configured as a stimulation contact to deliver electrical stimulation to tissue of a patient through a gated channel established by the array switch or configured as a collection contact to collect a physiological signal from tissue of a patient through a gated channel established by the array switch;
wherein the switch control signal is used for isolating the stimulation contact for delivering the electrical stimulation from the collection contact for collecting the physiological signal.

2. The implantable electrode of claim 1, wherein the switch control signal comprises a high-level signal and a low-level signal, the high-level signal is used for gating a channel of the array switch, and the low-level signal is used for disconnecting a channel of the array switch.

3. The implantable electrode of claim 1, wherein the array switch comprises a plurality of switch modules,
wherein each switch module comprises:
- an insulating layer configured to form an accommodation cavity;
- a switch unit disposed in the accommodation cavity formed by the insulating layer and configured to establish, according to the switch control signal, a gated channel of a contact corresponding to the switch control signal; and
- a conductive layer coating an outer surface of the insulating layer and configured to be grounded through a wire to shield at least one of electric-field interference or electromagnetic interference.

4. The implantable electrode of claim 1, wherein the electrode array module is a Utah electrode.

5. The implantable electrode of claim 1, wherein the physiological signal is a local field potential signal.

6. A stimulator, comprising:
- the implantable electrode of any one of claims 1 to 5, wherein the implantable electrode is configured to deliver the electrical stimulation to the tissue of the patient and collect the physiological signal from the tissue of the patient; and
- the pulse generator electrically connected to the implantable electrode directly or through an extension lead and configured to parse the physiological signal and generate the electrical stimulation.

7. The stimulator of claim 6, wherein the pulse generator comprises:
- a stimulation contact determination module configured to use at least one contact in the electrode array module as at least one stimulation contact according to therapy information of the patient, wherein each stimulation contact is configured to deliver the electrical stimulation to the tissue of the patient;
- an isolation contact acquisition module configured to acquire, for each stimulation contact, a plurality of contacts as isolation contacts, wherein the plurality of contacts satisfy at least one of a preset distance from the stimulation contact or a preset position relationship with the stimulation contact; and
- a collection contact determination module configured to select a contact from contacts other than the at least one stimulation contact and the isolation contacts of each stimulation contact according to the therapy information to serve as a collection contact; and, when the stimulation contact delivers the electrical stimulation to the tissue of the patient, collect the physiological signal from the tissue of the patient by using the collection contact and take the collected physiological signal as collection information.

8. The stimulator of claim 7, wherein the therapy information comprises a preset parameter corresponding to each stimulation contact, and the preset parameter indicates a distance relationship or a position relationship between the stimulation contact and the isolation contacts corresponding to the stimulation contact.

9. The stimulator of claim 8, wherein the isolation contact acquisition module comprises:
- a parameter prediction unit configured to input a stimulation parameter corresponding to the stimulation contact into a prediction model to acquire a reference preset parameter corresponding to the stimulation contact;
- a similarity determination unit configured to acquire a first similarity between the reference preset parameter and the preset parameter;
- a first contact acquisition unit configured to, in response to the first similarity being less than a preset similarity, perform an eight-neighborhood search on the stimulation contact based on the preset parameter to acquire the isolation contacts; and
- a second contact acquisition unit configured to, in response to the first similarity being not less than the preset similarity, update the preset parameter by using the reference preset parameter and perform the eight-neighborhood search on the stimulation contact based on the updated preset parameter to acquire the isolation contacts.

10. The stimulator of claim 9, wherein the pulse generator further comprises:
- a counting module configured to, in response to the first similarity being less than the preset similarity, add one to a count and determine whether the count added by one is greater than a preset count; and
- a risk prompt module configured to, in response to the count added by one being greater than the preset count, generate risk prompt information and clear the count added by one.

11. The stimulator of claim 7, wherein the pulse generator further comprises:
- a recommended-contact acquisition module configured to determine a score of each stimulation contact according to a stimulation parameter and collection information corresponding to each stimulation contact and use a stimulation contact with a highest score as a recommended stimulation contact.

12. A control method of a stimulator, comprising:
- using at least one contact in an electrode array module as at least one stimulation contact according to therapy information of a patient, wherein each stimulation contact is configured to deliver electrical stimulation to tissue of the patient;
- acquiring, for each stimulation contact, a plurality of contacts as isolation contacts, wherein the plurality of contacts satisfy at least one of a preset distance from the stimulation contact or a preset position relationship with the stimulation contact; and
- selecting a contact from contacts other than the at least one stimulation contact and the isolation contacts of each stimulation contact according to the therapy information to serve as a collection contact; and, when the stimulation contact delivers the electrical stimulation to the tissue of the patient, collecting a physiological signal from the tissue of the patient by using the collection contact and taking the collected physiological signal as collection information.

13. The control method of claim 12, wherein the therapy information comprises a preset parameter corresponding to each stimulation contact, and the preset parameter indicates a distance relationship or a position relationship between the stimulation contact and the isolation contacts corresponding to the stimulation contact.

14. The control method of claim 13, wherein acquiring, for each stimulation contact, the plurality of contacts as isolation contacts, wherein the plurality of contacts satisfy at least one of the preset distance from the stimulation contact or the preset position relationship with the stimulation contact, comprises:
- inputting a stimulation parameter corresponding to the stimulation contact into a prediction model to acquire a reference preset parameter corresponding to the stimulation contact;
- acquiring a first similarity between the reference preset parameter and the preset parameter;
- in response to the first similarity being less than a preset similarity, performing an eight-neighborhood search on the stimulation contact based on the preset parameter to acquire the isolation contacts; and
- in response to the first similarity being not less than the preset similarity, updating the preset parameter by using the reference preset parameter and performing the eight-neighborhood search on the stimulation contact based on the updated preset parameter to acquire the isolation contacts.

15. An electronic device, comprising a memory and a processor, wherein the memory is configured to store a computer program, and the processor is configured to, when executing the computer program, perform the control method of the stimulator of any one of claims 12 to 14.

16. A computer-readable storage medium storing a computer program which, when executed by a processor, causes the processor to perform the control method of the stimulator of any one of claims 12 to 14.
